# EUROPÄISCHE PATENTANMELDUNG

(11) **EP 3 888 537 A1**
(43) Veröffentlichungstag der Anmeldung: **06.10.2021**
(21) Anmeldenummer: 21165254.0
(22) Anmeldetag: 26.03.2021
(51) Int. Cl.: A61B 5/0205, A61B 5/0507, G16H 50/80, A61B 5/01, G01S 13/86, G01J 5/00, A61B 5/00, G07C 9/00

(54) **VORRICHTUNG UND VERFAHREN ZUR KONTAKTLOSEN MESSUNG ZUMINDEST EINES VITALPARAMETERS EINER PERSON**

(30) Priorität: 31.03.2020 DE 102020108973
(71) Anmelder: FRAUNHOFER-GESELLSCHAFT zur Förderung der angewandten Forschung e.V., 80686 München (DE)
(72) Erfinder: Schneider, Urs, 70569 Stuttgart (DE); Mannuß, Oliver, 70569 Stuttgart (DE); Huber, Marco, 70569 Stuttgart (DE); Chen, Xi, 70569 Stuttgart (DE); Aziz, Fady, 70569 Stuttgart (DE); Yaman, Alper, 70569 Stuttgart (DE)
(74) Vertreter: Lemcke, Brommer & Partner Patentanwälte Partnerschaft mbB

(57) **Zusammenfassung**

Die vorliegende Erfindung betrifft eine Vorrichtung (1) zur kontaktlosen Messung zumindest eines Vitalparameters zumindest einer Person (16, 16a), umfassend zumindest eine Radar-Einheit (2, 2a) zur Erfassung zumindest eines ersten Vitalparameters der Person (16, 16a), insbesondere einer Herzfrequenz, einer Atemfrequenz und/oder einer Amplitude einer Brustbewegung der Person (16, 16a), mittels Radar-Messungen. Weiter umfasst die Vorrichtung zumindest eine Thermographie-Einheit (3), insbesondere zur Erfassung eines weiteren Vitalparameters der Person (16, 16a), beispielsweise der Körpertemperatur, und eine Auswerteeinheit (4), welche mit der zumindest einen Radar-Einheit (2, 2a) und der Thermographie-Einheit (3) wirkverbunden ist, wobei mittels der Thermographie-Einheit (3) Mikro-Körperbewegungen der Person (16, 16a) erfassbar sind. Die Auswerteeinheit (4) ist weiter dazu ausgebildet, den erfassten ersten Vitalparameter in Abhängigkeit von den erfassten Mikro-Körperbewegungen zu korrigieren. Weiter betrifft die Erfindung ein Verfahren zur kontaktlosen Messung zumindest eines Vitalparameters zumindest einer Person (16, 16a).

## Beschreibung

Die vorliegende Anmeldung betrifft eine Vorrichtung zur kontaktlosen Messung von zumindest einem Vitalparameter zumindest einer Person nach dem Patentanspruch 1 sowie ein Verfahren zur kontaktlosen Messung von zumindest einem Vitalparameter von zumindest einer Person nach Patentanspruch 13.

Wie schnell sich neuartige Krankheiten zu einer weltweiten Pandemie entwickeln können zeigt sich bei der Verbreitung des neuartigen Coronavirus und der Covid-19-Pandemie deutlich. Eine pandemische Entwicklung von Krankheiten wird meist dadurch begünstigt, dass entsprechende Krankheiten erst sehr spät erkannt werden oder entsprechende Heilmittel nicht oder nicht in entsprechend großer Anzahl vorhanden sind. In solchen Fällen können daher meist nur Präventivmaßnahmen wie beispielsweise häufiges Händewaschen oder das Tragen einer Mund-Nasen-Bedeckung im Falle der Covid-19-Pandemie helfen sich und auch andere vor einer Ansteckung zu bewahren. Daneben können auch politische Maßnahmen wie beispielsweise Reisebeschränkungen, Grenzschließungen, die Anordnung von Quarantäne, Ausgangssperren oder das Schließen von Einrichtungen ergriffen werden, um eine weitere Verbreitung von Krankheiten zumindest einzudämmen.

Ein weiteres Mittel zur Begrenzung einer Verbreitung von Krankheitserregern kann über ein Abstandsgebot zwischen Personen unterschiedlicher Hausstände wie auch die Einschränkung sozialer Kontakte ("social distancing") erzielt werden. Allerdings ist dies bei Orten, an denen viele Menschen täglich zusammenkommen, wie beispielsweise an Flughäfen, in Bahnhöfen oder in öffentlichen Verkehrsmitteln, teils schwierig umzusetzen. Darüber hinaus möchte man den Zutritt zu bestimmten Bereichen oder Einrichtungen, wie beispielsweise Pflegeheime, Krankenhäuser oder Einrichtungen der öffentlichen Ordnung, auf solche Personen beschränken, welche mit ausreichend hoher Wahrscheinlichkeit keine Anzeichen für eine bestimmte Krankheit aufweisen.

So ist es beispielsweise schon bekannt über digitale Fieberthermometer die Körpertemperatur als ein Vitalparameter der Personen zu erfassen, wobei hierbei die Körpertemperatur beispielsweise an der Stirn oder im Ohr einer Person erfasst wird. Allerdings muss hierzu Personal eingesetzt werden, welches einerseits in Kontakt mit einer Vielzahl von Personen treten muss und somit einer potentiellen Gefahr ausgesetzt wird und andererseits auch Personalkosten entstehen.

Daneben gibt es bereits schon die Möglichkeit mittels Wärmebildkameras die Körpertemperatur von Personen annähernd zu erfassen, beispielsweise an Zugängen zu Gebäuden oder in Flughäfen, und gegebenenfalls Maßnahmen bei Feststellung einer erhöhten Körpertemperatur zu treffen. Die Nutzung von Wärmebildkameras ermöglicht eine kontaktlose Messung der Körpertemperatur über eine bestimmte Distanz hinweg, sodass eingesetztes Personal nicht einer potentiellen Gefahr ausgesetzt wird. Auch können derartige Systeme eine höhere Anzahl an Personen über einen Zeitraum erfassen, wodurch ebenfalls weniger Personal benötigt wird und Wartezeiten vermieden werden.

Darüber hinaus ist der Einsatz von Radar-Einheiten bekannt zur Bestimmung einer Position eines Objektes oder einer Person, insbesondere einer Entfernung der Person von der Radar-Einheit. Die Bestimmung erfolgt hierbei insbesondere über frequenzmodulierte Dauerstrichradar-Messungen. Beim frequenzmodulierten Dauerstrichradar (FMCW-Radar, engl. frequency modulated continuous wave radar) wird die Sendefrequenz zeitlich periodisch verändert. Auch ist es bereits bekannt, dass mittels Radar-Messungen Vitalparameter von Personen erfasst werden können.

Allerdings hat sich gezeigt, dass die Messungen von Vitalparametern eine Person oft zwar ein gutes, aber nicht immer ein hinreichend ausreichendes Ergebnis liefern. Dies hängt unter anderem damit zusammen, dass die zu messende Person sich meist bewegt und somit während der Messung unter anderem eine Distanzänderung zum Messgerät erfolgt. Es kann daher zur Überlagerung von verschiedenen Bewegungen kommen. Dies trifft insbesondere für Messung von Bewegungen des Körpers einer Person zu, beispielsweise bei der Messung der Atemfrequenz.

Daher ist es durchaus möglich, dass das erfasste Messergebnis nicht genau den eigentlichen Vitalparameter widerspiegelt und es somit zu Fehleinschätzungen in Bezug auf einen Gesundheitszustand einer Person auf Basis der erfassten Vitalparameter kommen kann. Schließlich können auch die Umgebungsbedingungen die Messung zusätzlich beeinflussen, sodass insgesamt bei der Erfassung eines Vitalparameters tatsächlich ein Messwert aus einer Vielzahl von Überlagerungen von verschiedenen Parametern erfasst wird. So kann es vorkommen, dass Personen trotz eines kritischen Vitalparameters nicht erkannt werden und somit oft unbewusst für eine weitere Verbreitung von Krankheitserregern sorgen.

Es ist daher die Aufgabe der vorliegenden Erfindung eine Vorrichtung und ein Verfahren zur kontaktlosen Messung von Vitalparametern zu schaffen, bei welchen die Erfassung zumindest eines ersten Vitalparameters verbessert wird.

Gelöst wird diese Aufgabe durch eine Vorrichtung zur kontaktlosen Messung von zumindest einem Vitalparameter gemäß Anspruch 1. Vorteilhafte Ausgestaltungen der Vorrichtungen finden sich in den abhängigen Ansprüchen 2 bis 12. Zudem wird die Aufgabe gelöst durch ein Verfahren zur kontaktlosen Messung von zumindest einem Vitalparameter gemäß Anspruch 13. Vorteilhafte Ausgestaltungen des Verfahrens finden sich in den abhängigen Ansprüchen 14 bis 15.

Die erfindungsgemäße Vorrichtung zur kontaktlosen Messung zumindest eines Vitalparameters ist bevorzugt zum Durchführen des erfindungsgemäßen Verfahrens ausgebildet, insbesondere einer vorteilhaften Ausgestaltung hiervon. Das erfindungsgemäße Verfahren zur kontaktlosen Messung zumindest eines Vitalparameters ist bevorzugt zur Durchführung mittels einer erfindungsgemäßen Vorrichtung, insbesondere einer vorteilhaften Ausgestaltungsform hiervon, ausgebildet.

Die erfindungsgemäße Vorrichtung zur kontaktlosen Messung von zumindest einem Vitalparameter zumindest einer Person umfasst zumindest eine Radar-Einheit zur Erfassung zumindest eines ersten Vitalparameters der Person, insbesondere einer Herzfrequenz, einer Atemfrequenz und/oder einer Amplitude der Brustbewegung, wobei die Erfassung mittels Radar-Messungen erfolgt. Weiter umfasst die erfindungsgemäße Vorrichtung zumindest eine Thermographie-Einheit, insbesondere zur Erfassung eines weiteren Vitalparameters der Person, beispielsweise der Körpertemperatur, wobei mittels der Thermographie-Einheit Mikro-Körperbewegungen der Person erfassbar sind, sowie eine Auswerteeinheit, welche mit der Radar-Einheit und der Thermographie-Einheit wirkverbunden ist. Weiter ist die Auswerteeinheit der erfindungsgemäßen Vorrichtung dazu ausgebildet, den erfassten ersten Vitalparameter in Abhängigkeit von den erfassten Mikro-Körperbewegungen zu korrigieren.

Vorzugsweise handelt es sich bei den Radar-Messungen um frequenzmodulierte Dauerstrichradar-Messungen mittels eines frequenzmodulierten Dauerstrichradars.

Bei den Mikro-Körperbewegungen der Person handelt es sich um kleine Bewegungen der Person, insbesondere um Schwankungen im Bereich des Oberkörpers der Person, insbesondere wenn die Person eigentlich in Ruhe auf einer Stelle steht. Diese Mikro-Körperbewegungen sind Bewegungen des Körpers der Person, welche diese dabei nicht unbedingt explizit ausführt und oft auch nicht selbst wahrnimmt. Es sind insbesondere unbewusste Schwankungen des Körpers mit geringer Amplitude. Die Amplitude solcher Schwankungen liegt insbesondere in einem Bereich kleiner 5 cm.

Bei den Vitalparametern der Person kann es sich insbesondere um die Herzfrequenz, Atemfrequenz oder Amplitude der Brustbewegung handeln. Weitere messbare und erfassbare Vitalparameter sind auch der Body-Mass-Index, die Lungenfunktion, beispielsweise um Lungeninfekte zu erkennen, oder die Sauerstoffsättigung der Person.

Durch die Korrektur des erfassten ersten Vitalparameters, welcher insbesondere die Herzfrequenz, die Atemfrequenz und/oder die Amplitude der Bewegung darstellen soll, in Abhängigkeit von den erfassten Mikro-Körperbewegungen der Person kann ein genauerer Wert für den ersten Vitalparameter angegeben werden. Hierdurch kann die Datenqualität des ersten Vitalparameters verbessert und eine genauere Einordnung des erfassten ersten Vitalparameters ermöglicht werden.

Beispielsweise bei der Bestimmung der Amplitude der Brustbewegung basiert der erfasste erste Vitalparameter ohne Korrektur auf der eigentlichen Bewegung der Brust der Person sowie einer Überlagerung durch die Mikro-Körperbewegungen. Erst durch die Korrektur kann der Messfehler durch die Überlagerung dieser Bewegungen korrigiert werden, so dass eine genauere Bestimmung des ersten Vitalparameters ermöglicht wird.

Eine Erfassung der Mikrokörperbewegungen erfolgt bevorzugt durch eine Analyse von mehreren mittels der Thermographie-Einheit erfassten Aufnahmen der Person, welche vorzugsweise unmittelbar nacheinander, insbesondere in Abständen von 0,8m bis 1,2m, aufgenommen wurden. Mit der Analyse der Aufnahmen ist eine Trajektorie der Person in einem bestimmten Zeitraum erfassbar. Für die Analyse werden die Aufnahmen insbesondere im Hinblick auf eine Änderung eines Augenabstands und/oder einer Änderung einer Körperoberfläche, vorzugsweise der Gesichtsfläche, ermittelt und aus der Änderung oder den Änderungen die Mikro-Körperbewegungen ermittelt. Insbesondere wird mittels der Thermographie-Einheit der optische Fluss von Bewegungen der Person detektiert, welcher durch die relative Bewegung der Person zur Thermographie-Einheit gegeben ist.

Vorzugsweise werden für die Mikro-Körperbewegungen nur die relativen Bewegungen der Person entlang einer Achse berücksichtigt, wobei die Achse durch eine Verbindungslinie zwischen Thermographie-Einheit und der Person gebildet wird. Bewegungskomponenten der Person senkrecht zu der Achse werden aus der ermittelten Trajektorie der Person für die Mikro-Körperbewegungen der Person in diesem Fall nicht berücksichtigt, da diese einen geringeren Einfluss auf die Erfassung des Vitalparameters haben können. Die Mikro-Körperbewegungen fließen als ein Kompensationswert in die Bestimmung des ersten Vitalparameters ein, wodurch eine entsprechende Korrektur erfolgt. Insbesondere bilden die Mikro-Körperbewegungen einen Offset für den ersten Vitalparameter.

Die erfindungsgemäße Vorrichtung erlaubt es kontaktlos eine Infektion bzw. zumindest den Verdacht auf eine Infektion oder eine andere Krankheit bei der Person festzustellen, wodurch gerade bei sehr ansteckenden Infektionen das Gesundheitsrisiko für alle minimiert wird. Durch die kontaktlose Messung von Vitalparametern wird insbesondere für Bediener der Vorrichtung bzw. entsprechendes Sicherheitspersonal das Infektions- und Gesundheitsrisiko reduziert, da diese einen entsprechend Abstand zu der Person einhalten kann. Zudem ermöglicht eine kontaktlose Messung mit der vorliegenden Vorrichtung gegenüber einer manuellen Messung durch eine automatische Erfassung der Vitalparameter einen höheren Personendurchsatz pro Zeiteinheit, wodurch einerseits Ansammlungen vor entsprechenden Stellen vermieden werden können und gleichzeitig auch der Einsatz an Bedienern bzw. Sicherheitspersonal reduziert werden kann. Auch wird durch die Vorrichtung der durch die notwendige Verwendung von Schutzausrüstung verursachte Müll für die Bediener bzw. Sicherheitspersonal reduziert.

Eine bevorzugte Ausführungsform zeichnet aus, dass die Vorrichtung zumindest zwei Radar-Einheiten, welche vorzugsweise in einer Ausstrahlrichtung einer Radar-Strahlung im Wesentlichen gegenüberliegend, insbesondere auf gleicher Höhe angeordnet sind, oder welche vorzugsweise nebeneinander und/oder versetzt zueinander, insbesondere auf einer Seite der Vorrichtung, angeordnet sind. Durch die Anordnung von zumindest zwei Radar-Einheiten kann die Messung des ersten Vitalparameters weiter verbessert werden. Insbesondere können die beiden Radar-Einheiten den ersten Vitalparameter unabhängig voneinander erfassen, wodurch der erfasste Vitalparameter verifiziert werden kann und die Datenqualität insgesamt erhöht werden kann. Insbesondere umfasst eine Anordnung nebeneinander sowohl eine Anordnung der zwei Radar-Einheiten horizontal als auch vertikal nebeneinander. Vorzugsweise umfasst eine Anordnung der zumindest zwei Radar-Einheiten versetzt zueinander, dass die Radar-Einheiten vertikal, horizontal und/oder radial zueinander versetzt angeordnet sind und insbesondere eine bestimmte Distanz zwischen den einzelnen Radar-Einheiten besteht.

Vorzugsweise kann die zweite Radar-Einheit zur Bestimmung der Bewegung des Körpers der Person verwendet werden.

Bevorzugt weist die Radar-Strahlung einer ersten Radar-Einheit eine erste Frequenz und/oder ein erstes moduliertes Signal auf und die Radar-Strahlung einer zweiten Radar-Einheit eine zweite Frequenz und/oder ein zweites moduliertes Signal. Vorzugsweise unterscheiden sich die erste Frequenz und/oder das erste modulierte Signal von der zweiten Frequenz und/oder dem zweiten modulierten Signal. Durch Radar-Strahlung der zumindest zwei Radar-Einheiten mit unterschiedlichen Frequenzen und/oder unterschiedlich modulierten Signalen wird es ermöglicht, dass jedes Signal bzw. jede Frequenz separat erfasst werden kann und somit eine Erfassung des ersten Vitalparameters auf Basis voneinander separater Datensätze erfolgen kann. Insbesondere kann durch die Verwendung von unterschiedlichen Frequenzen und/oder modulierten Signalen eine fehlerhafte Erfassung von Radar-Strahlung der ersten und/oder zweiten Radar-Einheit durch die zweite und/oder erster Radar-Einheit vermieden werden.

Vorzugsweise liegt die Frequenz der Radar-Strahlung in einem Bereich von 1 GHz bis 90 GHz, insbesondere bei 24 GHz oder 60 GHz.

Weiter bevorzugt ist die Radar-Einheit auf Basis eines SISO-Systems mit nur einer Antenne auf Senderseite und nur einem Empfänger auf Empfängerseite und/oder auf Basis eines MIMO-Systems mit jeweils mehreren Sendern auf Senderseite und mehreren Empfängern auf Empfängerseite ausgebildet.

In einer bevorzugten Ausführungsform ist die Radar-Einheit gegenüberliegend einer Wandfläche ausgebildet, welche die von der Radar-Einheit ausgestrahlte Radar-Strahlung zumindest teilweise, bevorzugt vollständig absorbiert oder diffus streut. Hierdurch kann eine Reflexion der von der Radar-Einheit ausgestrahlten Radar-Strahlung an, in Ausstrahlrichtung hinter der Person angeordneten Wandflächen zumindest reduziert oder gar vollständig unterbunden werden. Hierdurch kann die Radar-Messung weiter verbessert werden, da nur an der Person selbst eine Reflexion der Radar-Strahlung erfolgt und eine weitere störende Reflexion unterbunden werden kann. Damit kann die Erfassung des ersten Vitalparameters weiter verbessert werden.
Vorzugsweise erfolgt die Detektion der Person auf Basis der Clutter Methode.

Alternativ oder vorzugsweise ergänzend beträgt der horizontale oder radiale Abstand der Radar-Einheit und/oder der Thermographie-Einheit zu der Person 1 m bis 3 m, bevorzugt 1 m bis 1,5 m, besonders bevorzugt etwa 1 m. Durch den Abstand wird einerseits eine optimale Messung des ersten und ggf. des weiteren Vitalparameters ermöglicht und andererseits auch ein Abstand zu einem möglichen Bediener der Vorrichtung ausgebildet, wodurch dieser nicht direkt einem Gesundheitsrisiko ausgesetzt ist. Auch kann durch einen Abstand zwischen der Radar-Einheit und/oder der Thermographie-Einheit eine Beeinflussung oder Beschädigung dieser Einheiten reduziert oder vollständig unterbunden werden.

Insbesondere handelt es sich bei der Thermographie-Einheit um eine thermische Langwellen-Infrarotkamera. Mit der thermischen Langwellen-Infrarotkamera können die Mikro-Körperbewegungen besonders gut erfasst werden. Zudem eignet sich diese Infrarotkamera besonders zur Erfassung des weiteren Vitalparameters, insbesondere der Körpertemperatur der Person.

Die Körpertemperatur als weiterer Vitalparameter wird vorzugswiese mittels der Thermographie-Einheit im Gesicht der Person ermittelt, insbesondere zwischen den Augen der Person. Die Erfassung im Gesicht, insbesondere zwischen den Augen der Person, liefert eine hohe Genauigkeit der Messung. Alternativ oder vorzugsweise ergänzend kann die Messung auch im Mundbereich bei geöffnetem Mund oder im Ohr erfolgen. Durch Messung der Körpertemperatur kann insbesondere festgestellt werden, ob die Person Fieber hat, was als ein Zeichen einer Infektion gilt.

Alternativ oder vorzugsweise ergänzend ist zur Kalibrierung der Thermographie-Einheit ein Kalibrierelement, insbesondere der Thermographie-Einheit gegenüberliegend angeordnet. Vorzugsweise handelt es sich bei dem Kalibrierelement um einen temperierten Schwarzkörper. Durch das Kalibrierelement erfolgt in regelmäßigen oder unregelmäßigen Abständen eine Kalibrierung der Thermographie-Einheit, wodurch Messfehler reduziert und die Datenqualität der erfassten Vitalparameter weiter verbessert wird.

Eine bevorzugte Ausführungsform zeichnet aus, dass die zumindest eine RadarEinheit höhenverstellbar ist, insbesondere derart, dass diese auf die Brust der Person ausrichtbar ist. Alternativ oder vorzugsweise ergänzend ist die Thermographie-Einheit höhenverstellbar ausgebildet, insbesondere derart, dass diese auf das Gesicht der Person ausrichtbar ist. Durch die Höhenverstellung der Radar-Einheit und/oder der Thermographie-Einheit, welche separat voneinander oder zusammen erfolgen kann, können diese auf die jeweilige Person, insbesondere deren Position, Größe und Haltung, ausgerichtet werden und so Messfehler reduziert und die Qualität der erfassten Daten weiter verbessert werden.

Vorzugsweise umfasst die Vorrichtung eine erste Anzeigeeinheit, welche einem Bediener der Vorrichtung zugewandt und mit der Auswerteeinheit wirkverbunden ist, um auf einer Anzeigefläche der ersten Anzeigeeinheit zumindest den ersten Vitalparameter und insbesondere den weiteren Vitalparameter sowie bevorzugt weitere Daten darzustellen. Durch die Darstellung des zumindest einen ersten Vitalparameters auf der Anzeigefläche der ersten Anzeigeeinheit wird es dem Bediener der Vorrichtung ermöglicht, beispielsweise bei Überschreiten eines bestimmten Grenzwertes des ersten Vitalparameters, einzugreifen und die Person mit dem erhöhten Vitalparameter von den weiteren Personen, beispielsweise für detailliertere Untersuchungen, abzusondern.

Insbesondere kann die erste Anzeigeeinheit direkt an der Vorrichtung oder bevorzugt in einem Abstand zur Vorrichtung und zu der zu messenden Person angeordnet sein, sodass der Bediener einen entsprechend großen Abstand zu der Person aufweist.

Vorzugsweise ist die Vorrichtung mit einer Einhausung eingehaust, wodurch die Vorrichtung vor äußeren Umwelteinflüssen, beispielsweise Regen, wie auch vor Einwirkungen durch die Person oder durch Dritte geschützt ist.

Bevorzugt ist die Radar-Einheit separat abgedichtet.

Alternativ oder bevorzugt ergänzend umfasst die Vorrichtung weiter einen Schleusenraum, in welchen die Person zur kontaktlosen Erfassung des zumindest einen Vitalparameters eintritt. Der Schleusenraum kann hierbei eine Eingangsbeschränkung wie auch eine Ausgangsbeschränkung aufweisen, welche sich beispielsweise erst öffnen, wenn keine Person mehr im Schleusenraum ist bzw. die Messung des zumindest einen Vitalparameters erfolgreich war. Bei dem Schleusenraum kann es sich insbesondere um einen in sich abgeschlossenen Raum mit Eingang und Ausgang handeln, welcher bevorzugt eine eigene Lüftung, beispielsweise über einen Ventilator, aufweist. Insbesondere kann der Schleusenraum derart ausgebildet sein, dass darin stets gleichbleibende Umgebungsbedingungen herrschen. Insbesondere bevorzugt können die zumindest eine Radar-Einheit und/oder die zumindest eine Thermographie-Einheit außerhalb des Schleusenraums, unmittelbar am Schleusenraum, in einem Element des Schleusenraums oder im Schleusenraum selbst angeordnet sein.

In einer bevorzugten Ausführungsform ist zur Positionierung der Person eine Absperrung, insbesondere ein Zaun, angeordnet und/oder zumindest eine Markierung, insbesondere am Boden, ausgebildet. Durch die Absperrung und/oder zumindest eine Markierung wird die Person in einem für die Erfassung der Vitalparameter optimalen Abstand von der Radar-Einheit und/oder der Thermographie-Einheit platziert, so dass eine präzise Erfassung des zumindest einen Vitalparameters und insbesondere der Mikro-Körperbewegungen ermöglicht wird.

Vorzugsweise ist ein Temperatursensor zur Messung der Umgebungstemperatur und/oder ein Lichtsensor zur Messung der Lichtintensität der Umgebung der Person angeordnet, wobei die Auswerteeinheit bevorzugt dazu ausgebildet ist, die Umgebungstemperatur und/oder die Lichtintensität in die Erfassung der Mikro-Körperbewegungen und insbesondere des weiteren Vitalparameters mittels der Thermographie-Einheit einfließen zu lassen oder mit diesen zu verknüpfen. Hierdurch können Messfehler bei der Erfassung der Mikro-Körperbewegungen und insbesondere des weiteren Vitalparameters weiter reduziert werden. Durch Änderungen in der Umgebungstemperatur und/oder in der Lichtintensität der Umgebung der Person wird die Rate der Wärmeabgabe von der Körperoberfläche unmittelbar beeinflusst. Dies wirkt sich insbesondere auf den von der Thermographie-Einheit erfassten weiteren Vitalparameter aus, insbesondere die Körpertemperatur, da mittels der Thermographie-Einheit zunächst die Oberflächentemperatur der Person erfasst wird, welche jedoch in einer kalten Umgebung geringer sein kann als in einer warmen Umgebung. Durch das Erfassen der Umgebungstemperatur und/oder der Lichtintensität der Umgebung kann somit der weitere Vitalparameter genauer bestimmt werden.

Eine bevorzugte Ausführungsform der Vorrichtung zeichnet aus, dass die Vorrichtung dazu ausgebildet ist Vitalparameter von zumindest zwei Personen gleichzeitig zu erfassen, welche bevorzugt nebeneinander in einem Abstand von bis zu 1,5 m angeordnet sind. Der Abstand zwischen den Personen ergibt sich insbesondere aus der Distanz zwischen den gegenüberliegenden Schultern der Personen. Alternativ kann der Abstand auch als die Distanz zwischen den Körpermitten der Personen gesehen werden. Durch die gleichzeitige Erfassung von zumindest zwei Personen kann der Durchsatz der Vorrichtung erhöht werden, wodurch Staubildungen und größere Ansammlungen von Personen vor der Vorrichtung reduziert oder gar vermindert werden.

Eine bevorzugte Ausführungsform der Vorrichtung zeichnet aus, dass die Auswerteeinheit dazu ausgebildet ist, den zumindest einen erfassten weiteren Vitalparameter in Abhängigkeit von den Radar-Messungen zu korrigieren und/oder mit den Radar-Messungen zu verknüpfen, insbesondere zur Fokussierung der Thermographie-Einheit. Neben der Verknüpfung der Thermographie-Einheit mit der Radareinheit zur Korrektur des erfassten ersten Vitalparameters können auch die Radar-Messungen mit den erfassten Daten der Thermographie-Einheit verknüpft werden, sodass eine verbesserte Messgenauigkeit, insbesondere durch die Fokussierung der Thermographie-Einheit, erzielt werden kann.

Vorzugsweise umfasst die Vorrichtung eine zweite Anzeigeeinheit, deren Anzeigefläche der Person zugewandt und die mit der Auswerteeinheit wirkverbunden ist. Insbesondere handelt es sich bei der zweiten Anzeigeeinheit um ein Ampelsystem. Durch die zweite Anzeigeeinheit kann der Person das Ergebnis der Erfassung des zumindest einen Vitalparameters direkt angezeigt werden. Somit kann der Person unmittelbar angezeigt werden, ob sie beispielsweise Zutritt zu der Einrichtung bekommen kann oder ggf. einen Vitalparameter aufweist, wodurch Sie sich weiteren Untersuchungen unterziehen muss oder soll. Es empfiehlt sich hierbei nicht den unmittelbar erfassten Wert des Vitalparameters anzuzeigen, insbesondere aus Gründen der vereinfachten Zuordnung und Einordnung sowie aus Datenschutzgründen. Vielmehr ist es bevorzugt eine Kategorisierung vorzunehmen, beispielsweise in einem Ampelsystem, welches der Person das Ergebnis der Messung in vorgegebenen Kategorien anzeigt. Beispielsweise kann es sich hier um ein Ampelsystem mit den Kategorien "Weitergehen/Zugang" (Ampel auf Grün), "Wiederholung der Messung" (Ampel auf Orange) und "Zugang verwehrt" (Ampel auf Rot) handeln.

Weiter kann die Vorrichtung auch Zutrittsbeschränkungseinrichtungen, beispielsweise Türen, Schranken, oder ähnliches umfassen, welche mit der Auswerteeinheit wirkverbunden sind und sich beispielsweise erst öffnen, wenn kein kritischer Gesundheitszustand der Person erfasst wurde. Dies kann insbesondere in Einrichtungen mit erhöhten Sicherheitsvorkehrungen wie Flughäfen oder öffentlichen Einrichtungen Verwendung finden.

In einer bevorzugten Ausführungsform umfasst die Auswerteeinheit eine Datenbank für ein Klassifikationsschema zu einem Abgleich des zumindest einen Vitalparameters für eine Bestimmung und Klassifizierung eines Gesundheitszustands der Person. Durch ein Klassifikationsschema lassen sich somit die erfassten Vitalparameter einordnen und erlauben es, eine Entscheidung über das Passieren der Vorrichtung oder Maßnahmen wie beispielsweise ärztliche Untersuchungen, Absonderung oder Quarantäne zu ermöglichen.

Das Klassifikationsschema kann vorzugsweise auf den jeweiligen Einsatz, beispielsweise in einem Krankenhaus, an einem Flughafen oder bei einer Behörde, angepasst sein.

Vorzugsweise kann die Vorrichtung auch im Bereich des Sports eingesetzt werden, um Vitalparameter eines Sportlers, insbesondere nach einem absolvierten Training, zu ermitteln. Hierfür kann ein für diesen Einsatz abgestimmtes Klassifikationsschema bereitgestellt werden.

Bevorzugt kann die Vorrichtung auch im Falle einer Triage-Entscheidung ein spezielles Klassifikationsschema umfassen.

Bevorzugt umfasst die Auswerteeinheit eine Datenschnittstelle, die dazu ausgebildet ist die erfassten Daten zur weiteren Verarbeitung bereitzustellen und/oder weitere Daten zu empfangen. Insbesondere ermöglicht es eine Datenschnittstelle die von der Person erfassten Vitalparameter beispielsweise im Rahmen einer weiteren Untersuchung an einen Arzt weiterzuleiten oder den örtlichen Gesundheitsbehörden zur Verfügung zu stellen. Somit können die erfassten Vitalparameter als Ausgangspunkt für eine weitere Untersuchung der Person dienen.

Alternativ oder bevorzugt in Kombination zu den vorgenannten Merkmalen umfasst die Vorrichtung weiter eine Erfassungseinheit zur Erfassung zumindest der Identität der Person. Dies ermöglicht es den zumindest einen Vitalparameter eindeutig einer Person zuzuordnen. Auch wird durch eine Erfassung der Identität und weiterer Daten bei der Feststellung von kritischen Vitalparametern einer Person die Nachverfolgung erleichtert, insbesondere die Nachverfolgung von Infektionsketten. Vorzugsweise handelt es sich bei der Erfassungseinheit um eine Kamera und/oder einen Scanner, mittels welchem insbesondere ein Ausweisdokument oder ein Code, insbesondere ein QR-Code, erfassbar ist. Durch eine Kamera kann ein Bild der Person, insbesondere des Gesichtes aufgenommen werden und insbesondere die erfassten Vitalparameter dem Bild zugeordnet werden. Mittels Erfassen eines Ausweisdokuments können vorzugsweise die Sicherheitsvorkehrungen, beispielsweise bei Zutritt zu Behörden oder an Flughäfen weiter erhöht werden. Zudem können die erfassten Vitalparameter durch die weiteren Angaben im Ausweisdokument eindeutig zugeordnet werden. Eine Verschleierung von Personendaten kann somit unterbunden werden. Auch wird ein Abgleich des im Ausweisdokument vorhandenen Bildes mit einem erfassten Bild einer Kamera zur Identifizierung der ermöglicht.

Das Erfassen eines Codes, beispielsweise einer numerischen Zahlenfolge, ermöglicht es ebenfalls, dass die erfassten Vitalparameter eindeutig einer Person zugeordnet werden können, wobei durch die Verwendung eines Codes ein Grad der Anonymisierung erfolgen kann. Beispielsweise wird der Code durch eine Registrierung vorab unter Angaben gewisser Personendaten der Person zugeteilt.

Vorzugsweise werden die Daten lokal gespeichert und/oder an eine zentrale Leitstelle übermittelt um eine Nachverfolgung von Personen mit kritischen Vitalparametern zu ermöglichen.

Insbesondere können die Thermographie-Einheit und/oder der Lichtsensor und/oder der Temperatursensor über eine gemeinsame Schnittstelle mit der Auswerteeinheit verbunden sein. Bevorzugt erfolgt über die gemeinsame Schnittstelle neben einem Datentransfer auch die Stromversorgung der jeweiligen Einheiten und/oder Sensoren. Insbesondere handelt es sich bei der Schnittstelle um einen USB-Verteiler.

Die Radar-Einheit kann vorzugsweise über eine separate Stromversorgung aufweisen, welche eine Spannung von 5V und ein Strom von 5A bereitstellt.

Weiter wird die vorgenannte Aufgabe gelöst durch ein Verfahren zur Bestimmung von zumindest einem Vitalparameter zumindest einer Person, wobei mit einer Radar-Einheit zumindest ein erster Vitalparameter der Person, insbesondere eine Herzfrequenz, eine Atemfrequenz und/oder eine Amplitude der Brustbewegung, mittels Radar-Messungen erfasst wird. Weiter werden mit zumindest einer Thermographie-Einheit Mikro-Körperbewegungen der Person und vorzugsweise zumindest ein weiterer Vitalparameter der Person, beispielsweise eine Körpertemperatur, erfasst, wobei in einer Auswerteeinheit der zumindest eine erste Vitalparameter in Abhängigkeit von den erfassten Mikro-Körperbewegungen korrigiert wird.

Vorzugsweise handelt es sich bei den Radar-Messungen um frequenzmodulierte Dauerstrichradar-Messungen mittels eines frequenzmodulierten Dauerstrichradars.

Hierdurch wird eine Korrektur des erfassten, zumindest einen ersten Vitalparameters um die Mikro-Körperbewegungen ermöglicht, welche bei der Erfassung des ersten Vitalparameters zu dem eigentlichen Vitalparameter überlagert waren. Hierdurch wird das Messergebnis bzw. die Bestimmung des ersten Vitalparameters verbessert und somit die Datenqualität und seine Aussagekraft erhöht.

Eine bevorzugte Ausführungsform des Verfahrens zeichnet aus, dass in der Auswerteeinheit der zumindest eine weitere Vitalparameter in Abhängigkeit von den Radar-Messungen korrigiert und/oder mit den Radar-Messungen verknüpft wird. Insbesondere kann über die Radar-Messung ein Abstand der Person von der Vorrichtung bestimmt werden, wobei der über die Radar-Messungen erfasste Abstand zur Fokussierung der Thermographie-Einheit zur Erfassung von Mikro-Körperbewegungen und insbesondere eines weiteren Vitalparameters genutzt werden kann.

Vorzugsweise wird die Thermographie-Einheit mittels eines Kalibrierelements kalibriert, welches bevorzugt der Thermographie-Einheit gegenüberliegend angeordnet ist. Insbesondere kann es sich bei dem Kalibrierelement um einen temperierten Schwarzkörper handeln. Durch eine stetige Kalibrierung, welche in regelmäßigen oder unregelmäßigen Abständen erfolgen kann, wird sichergestellt, dass die von der Thermographie-Einheit erfassten Mikrokörperbewegungen und insbesondere der weitere Vitalparameter eine geringere Fehlertoleranz aufweisen.

Eine Kalibrierung der Thermographie-Einheit erfolgt bevorzugt vor jeder Inbetriebnahme der Vorrichtung, wobei eine Inbetriebnahme nach jeder Unterbrechung der Nutzung der Vorrichtung erfolgt. Alternativ kann eine Kalibrierung nach Ablauf eines bestimmten Zeitintervalls, beispielsweise nach 60 Minuten, oder nach einer bestimmten Anzahl an Messungen erfolgen.

Alternativ oder bevorzugt in Kombination fließt eine Umgebungstemperatur und/oder eine Lichtintensität in der Umgebung der Person in die Erfassung der Mikro-Körperbewegungen und insbesondere des weiteren Vitalparameters mittels der Thermographie-Einheit ein oder wird mit diesen verknüpft. Somit können die Umgebungsbedingungen bei der Erfassung der Mikro-Körperbewegungen und insbesondere des weiteren Vitalparameters berücksichtigt werden, wodurch die Datenqualität verbessert und Messfehler reduziert werden können. Auch können die Umgebungsbedingungen bei der Einordnung der erfassten Vitalparameter eine große Bedeutung einnehmen, beispielsweise bei einer sehr warmen Umgebungstemperatur oder der Kühlung des Schleusenraums mittels einer Klimaanlage.

Vorzugsweise wird der zumindest eine Vitalparameter über ein in einer Datenbank hinterlegtes Klassifikationsschema, welches in der Auswerteeinheit hinterlegt ist, klassifiziert und ein Gesundheitszustand der Person bestimmt und ausgegeben. Durch das Klassifikationsschema liegt es nicht unbedingt in der Verantwortung eines Bedieners zu entscheiden, ob die Person einen möglicherweise kritischen Gesundheitszustand aufweist. Vielmehr kann das Klassifikationsschema auf einer Vielzahl von Ergebnissen und Messungen und einer entsprechenden statistischen Auswertung und Gruppierung dieser Daten beruhen.

Eine weitere bevorzugte Ausführungsform des Verfahrens zeichnet aus, dass der zumindest eine Vitalparameter auf einer Anzeigefläche einer ersten Anzeigeeinheit einem Bediener dargestellt wird, sodass der Bediener bei Feststellung eines kritischen Vitalparameters entsprechend eingreifen kann. Beispielsweise kann die Person zur Durchführung von weiteren ärztlichen Untersuchungen oder zur Reduzierung eines Infektionsrisikos absondert werden.

Alternativ oder vorzugsweise ergänzend wird auf einer Anzeigefläche einer zweiten Anzeigeeinheit der Person eine Information auf Basis zumindest eines erfassten Vitalparameters visualisiert. Es kann hierbei die Anzeige des zumindest einen erfassten Vitalparameters erfolgen. Alternativ oder vorzugsweise ergänzend erfolgt eine Visualisierung auf Basis eines Ampelsystems. Durch die Anzeigefläche kann der Person eine unmittelbare Folge des erfassten Vitalparameters angezeigt werden, beispielsweise, dass die Person das Gebäude betreten darf, dass die Messung erneut durchgeführt wird oder dass ein Zutritt verwehrt wird.

Bevorzugt umfasst die Vorrichtung weiter eine Einrichtung zur berührungslosen Messung der Sauerstoffsättigung der Person und/oder eine Einrichtung zur Erstellung eines 3D-Scan der Person zum Erhalt von Informationen über deren Körpermaße und/oder Oberfläche, wobei die so ermittelten Daten oder Informationen vorzugsweise in die Auswerteeinheit mit einfließen.

Vorzugsweise umfasst die Vorrichtung zumindest ein Mikrofon zur Ermittlung von akustischen Informationen, welche bevorzugt in die Auswerteeinheit einfließen.

Weitere vorteilhafte Merkmale und Ausführungsformen der erfindungsgemäßen Vorrichtung sowie des erfindungsgemäßen Verfahrens werden im Folgenden anhand von Führungsbeispielen und den Figuren erläutert.

Es zeigen:
- Figur 1: eine erste Ausführungsform einer erfindungsgemäßen Vorrichtung in einer Seitenansicht;
- Figur 2: eine weitere Ausführungsform einer erfindungsgemäßen Vorrichtung;
- Figur 3: eine nochmals weitere Ausführungsform einer erfindungsgemäßen Vorrichtung in einer Draufsicht.

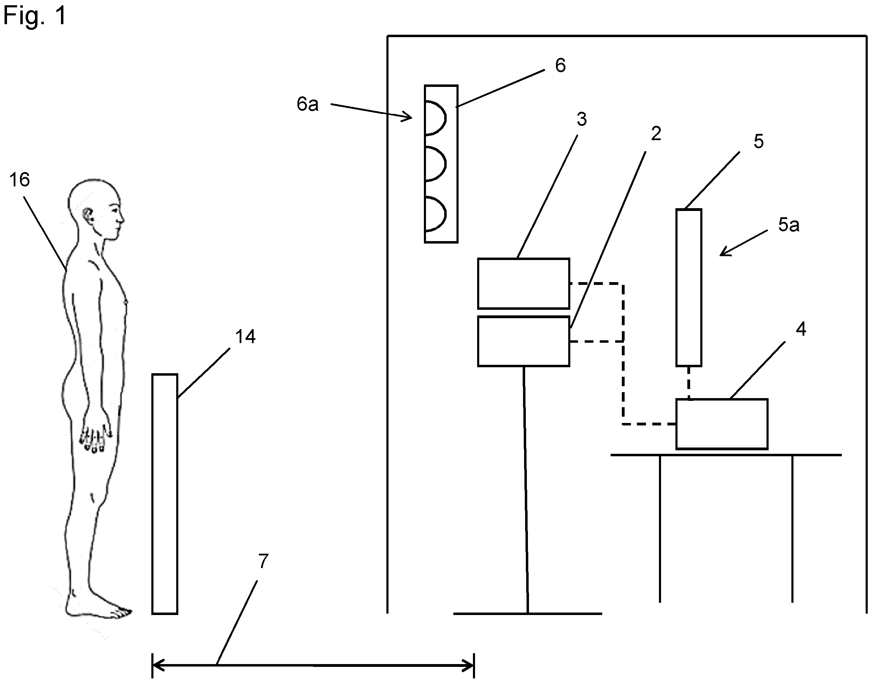

In den Figuren 1 bis 3 bezeichnen gleiche Bezugszeichen gleiche oder gleichwertige Elemente.

Figur 1 zeigt eine erste Ausführungsform einer erfindungsgemäßen Vorrichtung 1 zur kontaktlosen Messung zumindest eines Vitalparameters zumindest einer Person 16. Die Vorrichtung 1 umfasst eine Radar-Einheit 2 sowie eine Thermographie-Einheit 3, welche gemeinsam auf einem Stativ angeordnet sind. Über die Radar-Einheit 2 erfolgt eine Erfassung einer Atemfrequenz als ein erster Vitalparameter der Person 16 mittels Radar-Messungen, wobei es sich bevorzugt bei den Radar-Messungen um frequenzmodulierte Dauerstrichradar-Messungen handelt. Die Radar-Einheit 2 sowie die Thermographie-Einheit 3 sind hierbei auf eine Person 16 gerichtet, welche in einem horizontalen bzw. radialen Abstand 7 von etwa 1 m zu der Vorrichtung 1 steht.

Über die Thermographie-Einheit 3, welche als eine Langwellen-Infrarotkamera ausgebildet ist, werden einerseits Mikro-Körperbewegungen der Person 16 wie auch andererseits die Körpertemperatur als ein weiterer Vitalparameter der Person 16 erfasst.

Die Radar-Einheit 2 der Vorrichtung 1 ist dabei so angeordnet, dass diese auf die Brust der Person 16 gerichtet ist. Die Thermographie-Einheit 3 ist im Wesentlichen auf das Gesicht der Person 16 gerichtet, vorliegend auf den Bereich zwischen den Augen der Person 16. Der Bereich zwischen den Augen der Person 16 ermöglicht eine möglichst genaue Erfassung der Körpertemperatur der Person 16 als ein weiterer Vitalparameter.

Damit die Person 16 einen entsprechenden Abstand zur Vorrichtung 1 einhält, ist ein Absperrelement 14 in Form eines Zauns in einem Abstand 7 von der Radar-Einheit 2 bzw. der Thermographie-Einheit 3 angeordnet.

Die Radar-Einheit 2 wie auch die Thermographie-Einheit 3 sind weiter mit einer Auswerteeinheit 4 verbunden. In der Auswerteeinheit 4 werden die Daten bzw. Messergebnisse der Radar-Einheit 2 sowie der Thermographie-Einheit 3 zusammengeführt und einem Bediener der Vorrichtung 1 auf einer ersten Anzeigefläche 5a einer ersten Anzeigeeinheit 5 angezeigt.

Die Thermographie-Einheit 3 erfasst neben dem weiteren Vitalparameter in Form der Körpertemperatur auch Mikro-Körperbewegungen der Person 16, welche diese während der kontaktlosen Messung der Atemfrequenz meist unbewusst ausführt. Bei den Mikro-Körperbewegungen handelt es sich um kleinste Schwankungen des Körpers der Person 16 oder Bewegungen des Kopfes mit minimaler Amplitude.

Die Auswerteeinheit 4 der Vorrichtung 1 ist dabei so ausgebildet, die Atemfrequenz als den erfassten ersten Vitalparameter in Abhängigkeit von den erfassten Mikro-Körperbewegungen zu korrigieren. Die Atemfrequenz wird über eine Bewegung der Brust der Person 16 ermittelt, welche von den Mikro-Körperbewegungen der Person 16 überlagert wird. Der von der Radar-Einheit 2 erfasste Wert der Atemfrequenz entspricht somit einer Überlagerung einerseits der Bewegung der Brust der Person 16, aus welcher eine Atemfrequenz bestimmt wird, wie auch der Mikro-Körperbewegungen. Durch die Korrektur des erfassten Wertes für die Atemfrequenz in der Auswerteeinheit 4 um die Mikro-Körperbewegungen wird somit ein genauerer Wert für die Atemfrequenz der Person 16 ermittelt. Dies erlaubt es, dass auf Basis des Vitalparameters eine bessere Einschätzung über den Gesundheitszustand der Person 16 getroffen werden kann.

Über ein Klassifikationsschema, welches in einer Datenbank der Auswerteeinheit 4 umfasst ist, kann auf Basis der Atemfrequenz und Körpertemperatur als Vitalparameter ein potentieller Gesundheitszustand der Person 16 abgeleitet werden. Sofern die erfassten Vitalparameter in einem "Normalbereich" des Klassifikationsschemas liegen, kann die Person 16 die Vorrichtung 1 passieren und so beispielsweise Zugang zum Gebäude erlangen.

Sofern ein oder mehrere Vitalparameter der Person 16 oder deren Kombination in einem nach dem Klassifikationsschema kritischen Bereich liegen, wird dies einem Bediener der Vorrichtung 1 auf der Anzeigefläche 5a der Anzeigeeinheit 5 angezeigt. Der Bediener kann somit weitere Maßnahmen ergreifen. Die Person 16 kann beispielsweise von den anderen Personen abgesondert werden, um ein Infektionsrisiko zu minimieren. Auch besteht die Möglichkeit, dass sich die Person 16 weiteren ärztlichen Untersuchungen nach Feststellung von kritischen Vitalparametern durch die Vorrichtung 1 unterziehen muss.

Das Klassifikationsschema umfasst hierbei die Körpertemperatur, die Herzfrequenz, die Atemfrequenz und die Amplitude der Brustbewegung. Sofern die Körpertemperatur einen Grenzwert, vorliegend 37,5°C oder 38°C, und/oder die Herzfrequenz einen Grenzwert, vorliegend 90 oder 100 Schläge pro Minute, übersteigt, wird eine Warnung zur Untersuchung auf Fieber ausgegeben.

Sofern die Atemfrequenz einen Grenzwert, vorliegend 20 oder 30 Atemzüge pro Minute, übersteigt und die Amplitude der Brustbewegung einen Grenzwert, vorliegend kleiner 2 mm, unterschreitet, wird ebenfalls eine Warnung ausgegeben, mit dem Hinweis auf Untersuchung auf eine Lungenerkrankung.

Sofern alle vier vorgenannten Parameter den festgelegten Grenzwert überschreiten, sollte die Person 16 auf eine Infektion der Atemwege untersucht werden.

Ergänzend kann bei Ermittlung der Sauerstoffkonzentration eine Warnung bei Unterschreitung eines Grenzwerts, vorliegend kleiner 95%, allein oder in Kombination mit Grenzwerten der vorgenannten Parameter ausgegeben werden.

Die Auswerteeinheit 4 verfügt weiter über eine Datenschnittstelle, über welche Daten, wie beispielsweise ein aktualisiertes Klassifikationsschema, von der Auswerteeinheit 4 empfangen werden. Die Datenschnittstelle ermöglicht aber auch ein Bereitstellen und Weiterleiten der Vitalparameter einer Person 16. So kann bei Feststellung eines kritischen Vitalparameters ein Arzt bei einer weiteren Untersuchung der Person 16 auf die erfassten Vitalparameter zugreifen oder es können diese ihm zur Verfügung gestellt werden.

Die Vorrichtung 1 kann auch eine zweite Anzeigeeinheit 6 umfassen, deren Anzeigefläche 6a der Person 16 zugewandt ist. Bei der zweiten Anzeigeeinheit 6 handelt es sich vorliegend um eine Ampel mit den Ampelfarben Grün, Orange und Rot (von unten nach oben). Über die zweite Anzeigeeinheit 6 kann der Person 16 das Ergebnis der kontaktlosen Messung des Vitalparameters unmittelbar visualisiert werden. Wie auch von Ampeln aus dem Straßenverkehr bekannt, steht die Farbe Grün dafür, dass die kontaktlose Messung des Vitalparameters keine Auffälligkeiten zeigt und die Person 16 die Vorrichtung 1 passieren darf.

Sofern die Vorrichtung 1 einen kritischen Vitalparameter erfasst, kann dies auf der Anzeigefläche 6a der Anzeigeeinheit 6 durch ein rotes Signal signalisiert werden. Die Person 16 sollte sich von weiteren Personen isolieren und ohne Umwege durch einen Arzt weiter untersucht werden, um den Gesundheitszustand der Person 16 und eine potentielle Infektionsgefahr direkt zu ermitteln. Weiter kann ein oranges Ampelsignal auf der Anzeigefläche 6a der Anzeigeeinheit 6 der Person 16 anzeigen, dass die kontaktlose Messung von Vitalparametern nicht erfolgreich war und nochmals durchgeführt wird.

Ein Bediener der Vorrichtung 1 kann die kontaktlose Messung von Vitalparameter der Person 16 überwachen, hat jedoch einen ausreichenden Abstand zur Person 16, sodass ein Infektionsrisiko für den Bediener der Vorrichtung 1 gegenüber einer manuellen Messung eines Vitalparameters bei der Person 16 um ein Vielfaches reduziert wird.

Zum Schutz vor äußeren Umwelteinflüssen kann die Vorrichtung 1 weiter eine Einhausung 10 aufweisen, welche die Vorrichtung 1 vor direkter Sonneneinstrahlung, Regen oder Beeinflussung Dritter schützt. Die Einhausung 10 erlaubt es auch die Vorrichtung 1 im Freien zu verwenden und bei Bedarf vor entsprechenden Gebäuden oder Zugängen zu platzieren.

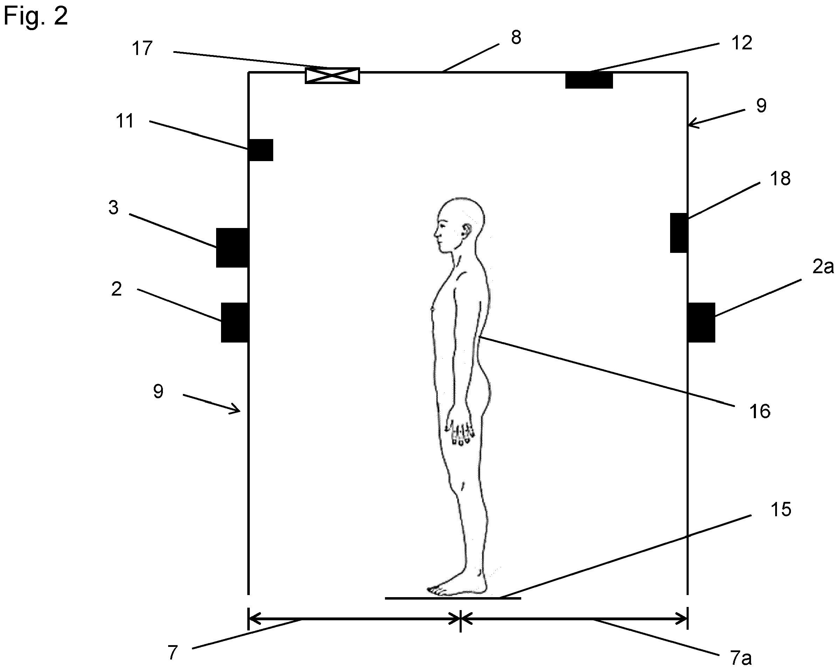

In Figur 2 ist eine weitere Ausführungsform der erfindungsgemäßen Vorrichtung 1 dargestellt, bei welcher die Person 16 zur kontaktlosen Messung der Herzfrequenz als Vitalparameter in einen Schleusenraum 8 eintritt, welcher entsprechende Wandflächen 9 aufweist.

Der Schleusenraum 8 ermöglicht es die kontaktlose Messung des zumindest einen Vitalparameters unter nahezu konstanten Bedingungen durchzuführen. Zudem ermöglicht der Schleusenraum 8 die Person 16 bei der Messung der Vitalparameter von weiteren Personen zu vereinzeln, sodass gezielt eine kontaktlose Messung des Vitalparameters nur an der jeweiligen im Schleusenraum 8 befindlichen Person 16 erfolgt.

Der Schleusenraum 8 weist einen Eingang auf, welcher sich erst öffnet, wenn keine Person 16 mehr im Schleusenraum 8 anwesend ist und dieser ggf. entsprechend desinfiziert oder belüftet wurde. Der Ausgang aus dem Schleusenraum 8 öffnet sich analog zum Ampelsystem als zweite Anzeigeeinheit 6, wie im Zusammenhang mit Figur 1 erläutert, nach erfolgter Klassifikation des Gesundheitszustands der Person 16 auf Basis der erfassten Vitalparameter.

Die in Figur 2 dargestellte Vorrichtung 1 umfasst weiter zwei Radar-Einheiten 2, 2a, welche an gegenüberliegenden Wandflächen 9 des Schleusenraums 8 angeordnet sind und im Wesentlichen auf die Brust und den Rücken der Person 16 gerichtet sind. Die Anordnung der Radar-Einheiten 2, 2a erfolgt auf gleicher Höhe über dem Boden des Schleusenraums 8. Der Schleusenraum 8 ist so dimensioniert, dass die Radar-Einheiten 2, 2a jeweils einen Abstand 7, 7a zu der Person 16 von etwa 1 m aufweisen. Die Wandflächen 9 des Schleusenraums 8 sind so ausgebildet, dass diese die von den Radar-Einheiten 2, 2a ausgestrahlte Radar-Strahlung absorbieren oder diffus streuen, sodass Messfehler auf Grund von an den Wandflächen 9 reflektierter Radar-Strahlung vermieden wird.

Die Radar-Strahlung der ersten Radar-Einheit 2 erzeugt hierbei eine Strahlung mit einer ersten Frequenz und einem ersten modulierten Signal. Die zweite Radar-Einheit 2a weist vorliegend eine Radar-Strahlung mit einer zur ersten Frequenz unterschiedlichen zweiten Frequenz auf, welche sich in der Modulation von der Radar-Strahlung der ersten Radar-Einheit 2 nicht unterscheidet. Hierdurch kann die Radar-Strahlung der jeweiligen Radar-Einheiten 2, 2a unterschieden werden, wodurch eine separate Erfassung der Radar-Strahlung durch die jeweilige Radar-Einheit 2, 2a erfolgen kann. Dies ermöglich eine Erfassung des ersten Vitalparameters durch die Radar-Einheiten 2, 2a getrennt voneinander und einen entsprechenden Abgleich der erfassten Daten.

Weiter ist eine Thermographie-Einheit 3 zur Erfassung der Mikro-Körperbewegungen und der Körpertemperatur der Person 16 in oder an einer Wandfläche 9 des Schleusenraums 8 angeordnet. Auf der der Thermographie-Einheit 3 gegenüberliegenden Wandfläche 9 ist weiter ein Kalibrierelement 18 zur Kalibrierung der Thermographie-Einheit 3 angeordnet. Bei dem Kalibrierelement 18 handelt es sich um einen temperierten Schwarzkörper, welcher eine bestimmte Temperatur aufweist. Zur Kalibrierung der Thermographie-Einheit 3 wird diese auf das Kalibrierelement 18 gerichtet und die mit der Thermographie-Einheit 3 gemessene Temperatur mit der Temperatur des temperierten Schwarzkörpers als Kalibrierelement 18 verglichen. Eine Kalibrierung der Thermographie-Einheit 3 erfolgt vor jeder Inbetriebnahme der Vorrichtung, wobei eine Inbetriebnahme nach jeder längeren Unterbrechung der Nutzung der Vorrichtung erfolgt.

Zur Erfassung der Umgebungstemperatur ist im Schleusenraum 8 weiter ein Temperatursensor 11 angeordnet. Die Umgebungstemperatur beeinflusst ebenfalls das Messergebnis der Thermographie-Einheit 3. So fließt über den Temperatursensor 11 die Umgebungstemperatur in die von der Thermographie-Einheit 3 erfassten Mikro-Körperbewegungen oder die Körpertemperatur als Vitalparameter ein bzw. es erfolgt eine Verknüpfung der erfassten Umgebungstemperatur mit den von der Thermographie-Einheit 3 erfassten Daten.

Weiter ist auch ein Lichtsensor 12 im Schleusenraum 8 zur Erfassung der Lichtintensität der Umgebung der Person 16 angeordnet, da die Lichtintensität ebenfalls die Messungen der Thermographie-Einheit 3 beeinflusst. Daher fließen auch die Messungen des Lichtsensors 12 zur Lichtintensität in die von der Thermographie-Einheit 3 erfassten Daten, wie Mikro-Körperbewegungen oder Körpertemperatur der Person 16, ein oder werden mit diesen verknüpft.

Zur Erfüllung hygienischer Anforderungen umfasst der Schleusenraum 8 eine aktive Belüftung 17 über einen Ventilator. Durch eine aktive Belüftung 17 erfolgt regelmäßig ein Luftaustausch im Schleusenraum 8, sodass ein potentielles Infektionsrisiko für die Person 16 durch in der Luft befindliche Krankheitserreger auf ein Minimum reduziert wird.

Damit sich die Person 16 im Schleusenraum 8 für eine korrekte kontaktlose Messung der Vitalparameter entsprechend platziert, sind auf dem Boden des Schleusenraums 8 Markierungen 15 angebracht, welche die Person 16 in die richtige Position leiten soll.

Ein Bediener der Vorrichtung 1 befindet sich außerhalb des Schleusenraums 8 und beobachtet die kontaktlose Messung von Vitalparametern. Sofern die Vorrichtung 1 einen kritischen Gesundheitszustand der Person 16 ausgibt, kann der Bediener entsprechend eingreifen und die Person mit dem kritischen Gesundheitszustand von weiteren Personen absondern und beispielsweise zu einer direkten Untersuchung durch einen Arzt begleiten. Auch kann die Person 16 bei Feststellung eines kritischen Gesundheitszustands unmittelbar nach Hause geschickt werden, beispielsweise unter der Auflage sich für einen bestimmten Zeitraum in Quarantäne zu begeben.

Die Radar-Messungen der Radar-Einheit 2, 2a ermöglichen neben der Erfassung des ersten Vitalparameters auch eine Bestimmung des Abstands 7. 7a der Person 16 von der Radar-Einheit 2, 2a. Die Radar-Messungen werden zudem mit den Messungen der Thermographie-Einheit 3 verknüpft. Die Bestimmung des Abstands 7, 7a durch die Radar-Messung mittels der Radar-Einheit 2, 2a ermöglicht die Einstellung des Fokus der Thermographie-Einheit 3.

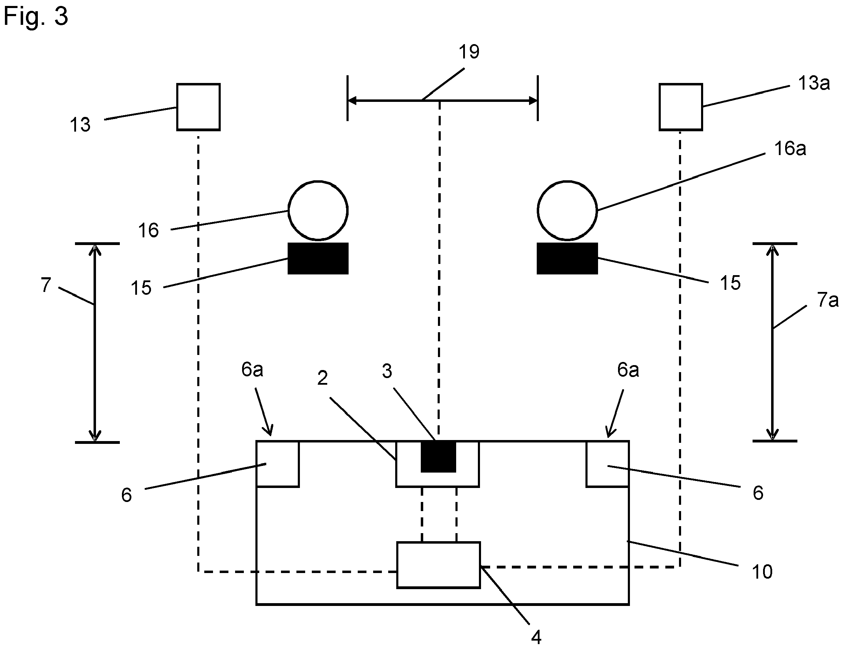

In Figur 3 ist in einer Draufsicht eine weitere Ausgestaltungsform mit der erfindungsgemäßen Vorrichtung 1 dargestellt, bei welcher bei zwei Personen 16, 16a gleichzeitig eine kontaktlose Messung von Vitalparametern mit einer Vorrichtung 1 erfolgt. Die Vorrichtung 1 weist wiederum, wie zuvor zu Figur 1 ausgeführt, eine Radar-Einheit 2, eine Thermographie-Einheit 3 sowie eine Auswerteeinheit 4 auf, welche in einer Einhausung 10 angeordnet sind. Die Personen 16, 16a stehen in einem Abstand 7, 7a von der Vorrichtung 1 von etwa 1,5 m. Der Abstand 19 zwischen den beiden Personen 16, 16a in der Vorrichtung 1 beträgt ebenfalls etwa 1,5 m, wobei sich der Abstand 19 auf die Distanz zwischen den gegenüberliegenden Schultern der Personen 16, 16a bezieht.

Die Vorrichtung 1 umfasst weiter zwei zweite Anzeigeeinheiten 6, um das Ergebnis der kontaktlosen Messung des Vitalparameters der jeweiligen Person 16, 16a auf den entsprechenden Anzeigeflächen 6a, welche als eine Ampel ausgebildet sind, anzeigen zu können.

Die Radar-Einheit 2 sowie die Thermographie-Einheit 3 sind jeweils so ausgebildet, dass diese beide Personen 16, 16a erfassen und aufgrund der versetzten Anordnung der Personen 16, 16a die Messungen eindeutig zuordnen können. Hierzu ist die Radar-Einheit auf Basis eines MIMO-Systems mit jeweils mehreren Sendern auf Senderseite und mehreren Empfängern auf Empfängerseite ausgebildet. Zur richtigen Positionierung der Person 16, 16a vor der Radar-Einheit 2 und der Thermographie-Einheit 3 sind auf dem Boden wiederum Markierungen 15 ausgebildet.

Daneben umfasst die Vorrichtung 1 weiter für die beiden Bereiche zur kontaktlosen Messung von Vitalparametern jeweils eine Erfassungseinheit 13, 13a, mittels welcher die Identität der jeweiligen Person 16, 16a erfasst wird. An der Erfassungseinheit 13, 13a wird das Ausweisdokument der Person 16, 16a gescannt. Die Erfassungseinheit 13, 13a ist mit der Auswerteeinheit 4 wirkverbunden, sodass ein erfasster Vitalparameter eindeutig der jeweiligen Person 16, 16a zugeordnet werden kann. Hierdurch wird insbesondere bei Zugang zu Sicherheitsbereichen an einem Flughafen oder bei Behörden die Sicherheit erhöht. Weiter kann eine Kamera in der Vorrichtung angeordnet sein, über welche eine Aufnahme des Gesichtes der Person 16, 16a erfolgt, wodurch ein Abgleich zwischen den erfassten Daten im Ausweisdokument, insbesondere dem Bild, mit einem aufgenommenen Bild der aktuell gemessenen Person 16, 16a ermöglicht wird.

### Bezugszeichenliste

- 1: Vorrichtung
- 2, 2a: Radar-Einheit
- 3: Thermographie-Einheit
- 4: Auswerteeinheit
- 5: Erste Anzeigeeinheit
- 5a: Anzeigefläche
- 6: Zweite Anzeigeeinheit
- 6a: Anzeigefläche
- 7, 7a: Abstand
- 8: Schleusenraum
- 9: Wandfläche
- 10: Einhausung
- 11: Temperatursensor
- 12: Lichtsensor
- 13, 13a: Erfassungseinheit
- 14: Absperrung
- 15: Markierung
- 16, 16a: Person
- 17: Lüftung
- 18: Kalibrierelement
- 19: Abstand

## Patentansprüche

1. Vorrichtung (1) zur kontaktlosen Messung zumindest eines Vitalparameters zumindest einer Person (16, 16a), umfassend
zumindest eine Radar-Einheit (2, 2a) zur Erfassung zumindest eines ersten Vitalparameters der Person (16, 16a), insbesondere einer Herzfrequenz, einer Atemfrequenz und/oder einer Amplitude einer Brustbewegung der Person (16, 16a), mittels Radar-Messungen,
zumindest eine Thermographie-Einheit (3), insbesondere zur Erfassung eines weiteren Vitalparameters der Person (16, 16a), beispielsweise der Körpertemperatur,
und eine Auswerteeinheit (4), welche mit der zumindest einen Radar-Einheit (2, 2a) und der Thermographie-Einheit (3) wirkverbunden ist, wobei mittels der Thermographie-Einheit (3) Mikro-Körperbewegungen der Person (16, 16a) erfassbar sind, und
wobei die Auswerteeinheit (4) dazu ausgebildet ist, den erfassten ersten Vitalparameter in Abhängigkeit von den erfassten Mikro-Körperbewegungen zu korrigieren.

2. Vorrichtung (1) nach Anspruch 1,
**dadurch gekennzeichnet,**
**dass** die Vorrichtung (1) zumindest zwei Radar-Einheiten (2, 2a) umfasst, welche vorzugsweise in einer Ausstrahlrichtung (19) einer Radar-Strahlung im Wesentlichen gegenüberliegend, insbesondere auf gleicher Höhe, angeordnet sind oder welche vorzugsweise nebeneinander und/oder versetzt zueinander, insbesondere auf einer Seite der Vorrichtung (1), angeordnet sind.

3. Vorrichtung (1) nach Anspruch 2,
**dadurch gekennzeichnet,**
**dass** die Radar-Strahlung einer ersten Radar-Einheit (2, 2a) eine erste Frequenz und/oder ein erstes moduliertes Signal aufweist und die Radar-Strahlung einer zweiten Radar-Einheit (2, 2a) eine zweite Frequenz und/oder ein zweites moduliertes Signal aufweist, wobei vorzugsweise die erste Frequenz und/oder das erste modulierte Signal sich von der zweiten Frequenz und/oder dem zweiten modulierten Signal unterscheidet.

4. Vorrichtung (1) nach einem der vorhergehenden Ansprüche,
**dadurch gekennzeichnet,**
**dass** der Radar-Einheit (2, 2a) gegenüberliegend eine Wandfläche (9) ausgebildet ist, welche die von der Radar-Einheit (2, 2a) ausgestrahlte Radar-Strahlung zumindest teilweise, bevorzugt vollständig absorbiert oder diffus streut und/oder
**dass** ein Abstand (7, 7a) der Radar-Einheit (2, 2a) und/oder der Thermographie-Einheit (3) zu der Person (16, 16a) 1 m bis 3 m, bevorzugt 1 m bis 1,5 m, besonders bevorzugt etwa 1 m beträgt.

5. Vorrichtung (1) nach einem der vorhergehenden Ansprüche,
**dadurch gekennzeichnet,**
**dass** die Thermographie-Einheit (3) eine thermische Langwellen-Infrarotkamera ist und/oder
**dass** zur Kalibrierung der Thermographie-Einheit (3) eine Kalibrierelement (18) angeordnet ist, insbesondere der Thermographie-Einheit (3) gegenüberliegend, wobei bevorzugt das Kalibrierelement (18) als ein temperierter Schwarzkörper ausgebildet ist.

6. Vorrichtung (1) nach einem der vorhergehenden Ansprüche,
**dadurch gekennzeichnet,**
**dass** die Radar-Einheit (2, 2a) höhenverstellbar ist, insbesondere derart, dass diese auf die Brust der Person (16, 16a) ausrichtbar ist und/oder dass die Thermographie-Einheit (3) höhenverstellbar ist, insbesondere derart, dass diese auf das Gesicht der Person (16, 16a) ausrichtbar ist.

7. Vorrichtung (1) nach einem der vorhergehenden Ansprüche,
**dadurch gekennzeichnet,**
**dass** die Vorrichtung (1) eine erste Anzeigeeinheit (5) umfasst, welche einem Bediener der Vorrichtung (1) zugewandt und mit der Auswerteeinheit (4) wirkverbunden ist, um auf einer ersten Anzeigefläche (5a) zumindest den ersten Vitalparameter darzustellen.

8. Vorrichtung (1) nach einem der vorhergehenden Ansprüche,
**dadurch gekennzeichnet,**
**dass** die die Vorrichtung (1) mittels einer Einhausung (10) eingehaust ist und/oder
**dass** die Vorrichtung (1) weiter einen Schleusenraum (8) umfasst, in welchen die Person (16, 16a) zur Bestimmung des zumindest einen Vitalparameters eintritt und/oder
**dass** zur Positionierung der Person (16, 16a) eine Absperrung (14), insbesondere ein Zaun, angeordnet und/oder zumindest eine Markierung (15), bevorzugt am Boden, ausgebildet ist.

9. Vorrichtung (1) nach einem der vorhergehenden Ansprüche,
**dadurch gekennzeichnet,**
**dass** ein Temperatursensor (11) zur Messung der Umgebungstemperatur und/oder ein Lichtsensor (12) zur Messung der Lichtintensität der Umgebung der Person (16) angeordnet ist,
wobei die Auswerteeinheit (4) bevorzugt dazu ausgebildet ist, die Umgebungstemperatur und/oder die Lichtintensität in die Erfassung der MikroKörperbewegungen und insbesondere des weiteren Vitalparameters mittels der Thermographie-Einheit (3) einfließen zu lassen oder mit diesen zu verknüpfen.

10. Vorrichtung (1) nach einem der vorhergehenden Ansprüche,
**dadurch gekennzeichnet,**
**dass** die Vorrichtung (1) dazu ausgebildet ist Vitalparameter von zumindest zwei Personen (16, 16a) zu erfassen, welche bevorzugt nebeneinander in einem Abstand von bis zu 1,5 m angeordnet sind und/oder dass die Vorrichtung (1) eine zweite Anzeigeeinheit (6) umfasst, deren Anzeigefläche (6a) der Person (16, 16a) zugewandt und die mit der Auswerteeinheit (4) wirkverbunden ist, insbesondere, dass es sich bei der zweiten Anzeigeeinheit (6) um ein Ampelsystem handelt und/oder dass die Vorrichtung (1) weiter eine Erfassungseinheit (13) zur Erfassung der Identität der Person (16, 16a) umfasst, bevorzugt eine Kamera und/oder einen Scanner, insbesondere zur Erfassung eines Ausweisdokumentes oder eines Codes.
**dass** die Vorrichtung (1) weiter eine Erfassungseinheit (13) zur Erfassung der Identität der Person (16, 16a) umfasst, bevorzugt eine Kamera und/oder einen Scanner, insbesondere zur Erfassung eines Ausweisdokumentes oder eines Codes.

11. Vorrichtung (1) nach einem der vorhergehenden Ansprüche,
**dadurch gekennzeichnet,**
**dass** die Auswerteeinheit (4) dazu ausgebildet ist, den weiteren Vitalparameter in Abhängigkeit von den Radar-Messungen zu korrigieren und/oder mit den Radar-Messungen zu verknüpfen, insbesondere zur Fokussierung der Thermographie-Einheit (3).

12. Vorrichtung nach einem der vorhergehenden Ansprüche,
**dadurch gekennzeichnet,**
**dass** die Auswerteeinheit (4) eine Datenbank für ein Klassifikationsschema umfasst zu einem Abgleich des zumindest einen Vitalparameters für eine Bestimmung eines Gesundheitszustands der Person (16, 16a) und/oder
**dass** die Auswerteeinheit (4) eine Datenschnittstelle umfasst, welche dazu ausgebildet ist die erfassten Daten zur weiteren Verarbeitung bereitzustellen und/oder weitere Daten zum empfangen.

13. Verfahren zur Bestimmung von Vitalparametern zumindest einer Person (16, 16a), wobei mit einer Radar-Einheit (2, 2a) zumindest ein erster Vitalparameter der Person (16), insbesondere eine Herzfrequenz, eine Atemfrequenz und/oder eine Amplitude der Brustbewegung, mittels Radar-Messungen, erfasst wird,
wobei mit zumindest einer Thermographie-Einheit (3) Mikro-Körperbewegungen der Person (16, 16a) und vorzugsweise ein weiterer Vitalparameter der Person (16), beispielsweise eine Körpertemperatur, erfasst werden, und
wobei in einer Auswerteeinheit (4) der erfasste, zumindest eine erste Vitalparameter in Abhängigkeit von den erfassten Mikro-Körperbewegungen korrigiert wird.

14. Verfahren nach Anspruch 13,
**dadurch gekennzeichnet,**
**dass** in der Auswerteeinheit (4) der weitere Vitalparameter in Abhängigkeit von den Radar-Messungen korrigiert und/oder mit den Radar-Messungen verknüpft wird und/oder
**dass** die Thermographie-Einheit (3) über ein Kalibrierelement (18), welches bevorzugt der Thermographie-Einheit (3) gegenüberliegend angeordnet ist, kalibriert wird, wobei das Kalibrierelement (18) insbesondere als ein temperierter Schwarzkörper ausgebildet ist, und/oder dass eine Umgebungstemperatur und/oder eine Lichtintensität in der Umgebung der Person (16, 16a) in die Erfassung der Mikro-Körperbewegungen und insbesondere des weiteren Vitalparameters mittels der Thermographie-Einheit (3) einfließen oder mit diesen verknüpft werden.

15. Verfahren nach einem der Ansprüche 13 oder 14,
**dadurch gekennzeichnet,**
**dass** der zumindest eine Vitalparameter über ein in einer Datenbank hinterlegtes Klassifikationsschema klassifiziert und ein Gesundheitszustand der Person (16, 16a) bestimmt und ausgegeben wird und/oder dass der zumindest eine Vitalparameter auf einer Anzeigefläche (5a) einer ersten Anzeigeeinheit (5) einem Bediener dargestellt wird und/oder auf einer Anzeigefläche (6a) einer zweiten Anzeigeeinheit (6) der Person (16, 16a) Befehle auf Basis des zumindest einen Vitalparameters visualisiert werden, insbesondere in Form eines Ampelsystems.
